# EUROPEAN PATENT SPECIFICATION

(11) **EP 1 070 043 B1**
(45) Date of publication and mention of the grant of the patent: **18.02.2004**
(21) Application number: 99915908.0
(22) Date of filing: 09.04.1999
(51) Int. Cl.: C07C 67/303, C07C 69/34, C07C 69/608

(54) **ASYMMETRIC HYDROGENATION**
ASYMMETRISCHE HYDRIERUNG
HYDROGENATION ASYMETRIQUE

(30) Priority: 09.04.1998 GB 9807888; 16.12.1998 WO PCT/GB98/03784
(43) Date of publication of application: 24.01.2001
(73) Proprietor: Chirotech Technology Limited, Cambridge CB4 0WG (GB)
(72) Inventor: BURK, Mark Joseph Chirotech Technology Limited, Cambridge CB4 0WG (GB); BIENEWALD, Frank, F-78000 Versailles (FR); ZANOTTI-GEROSA, Antonio, Milton Road Cambridge CB4 0WG (GB)
(74) Representative: Raynor, John
(86) International application number: PCT/GB1999/001103
(87) International publication number: WO 1999/052852

(56) References cited:
- WO-A-97/18894
- US-A- 4 939 288
- KEIJI YAMAMOTO ET AL.: "Optimization of asymmetric hydrogenation of 3-phenyl-3-butenoic acid catalyzed by rhodium(I)-4,5-bis((diphenylphosphino)meth yl)-2,2-dimethyldioxolane(DIOP)" JOURNAL OF ORGANOMETALLIC CHEMISTRY., vol. 370, no. 1-3, 11 July 1989 (1989-07-11), pages 319-332, XP002109270 ELSEVIER-SEQUOIA S.A. LAUSANNE., CH ISSN: 0022-328X cited in the application

## Description

### Field of the Invention

This invention relates to processes suitable for the large scale preparation of enantiomerically enriched chiral carboxylic acid derivatives. In particular, it relates to asymmetric hydrogenation of prochiral substrates, using a transition metal catalyst complex.

### Background of the Invention

Asymmetric hydrogenation has been used to convert prochiral substrates having the partial formula C=C-C-COOX to chiral compounds of the formula C-C-C-COOX. See, for example, Yamamoto et al, *J. Organometallic Chem*., **1989,** 370, 319, where the substrate is 3-phenyl-3-butenoic acid, and the catalyst is Rh-DIOP. X depends on the additive, including tertiary amines.

Examples of other substrates in such a reaction have generally had a carboxylate function at at least one chiral centre. For example, itaconic acid derivatives have been used.

Enantiomerically enriched 2-substituted succinic acids, have recently attracted interest as useful chiral building blocks and peptidomimetics in the design of pharmaceuticals, flavours and fragrances, and agrochemicals with improved properties. For example, the utility of 2-substituted succinic acid derivatives has been amply demonstrated through the synthesis of a range of new potent orally bioavailable drugs [J.J. Talley et al., in *Catalysis of Organic Reactions*, J.R. Kosak, T.A. Johnson (eds.), Marcel Dekker, Inc. (**1994**) Chapter 6; and H. Jendralla, *Synthesis* (**1994**) 494].

Chiral succinates can be prepared simply (e.g., via Stobbe condensation) from unsubstituted succinic esters and aldehydes or ketones, followed by asymmetric hydrogenation of the intermediate β-substituted itaconate derivatives. For example, itaconic acid or its sodium salt, can be enantioselectively hydrogenated to 2-methylsuccinic acid with rhodium catalysts bearing the chiral ligand N-acyl-3,3'-bis(diphenylphosphino)pyrrolidine (BPPM) in up to 92% enantiomeric excess (ee) [I. Ojima et al., *Chem. Lett*., **1978**, 567; I. Ojima et al., *Chem. Lett*., **1978**, 1145; K. Achiwa, *Tetrahedron Lett*., **1978**, 1475]. A rhodium catalyst bearing the chiral diphosphine DIP AMP affords 2-methylsuccinate in up to 88 % ee [W. C. Christofel, B. D. Vineyard, *J. Am. Chem. Soc*. **1979**, *101*, 4406; and US-A-4,939,288]. Similar results have been obtained with a ruthenium catalyst containing the chiral diphosphine ligand BINAP [H. Kawano et al., *Tetrahedron Lett*., **1987,** *28*, 1905]. Rhodium catalysts bearing modified DIOP ligands provide 2-methylsuccinic acid derivatives with variable enantioselectivities, between 7 and 91% ee. In these latter reactions, the ee value is very dependant on the rhodium catalyst precursor and whether the free acid or the ester is used [T. Morimoto et al., *Tetrahedron Lett*., **1989,** *30*, 735]. Better results have been reported with a neutral rhodium catalyst ofthe chiral diphosphine 2,2'-bis(dicyclohexylphosphino)-6,6'-dimethyl-1,1'-biphenyl (BICHEP), whereby dimethylitaconate was hydrogenated in 99% ee [T. Chiba et al., *Tetrahedron Lett*., **1991**, *32*, 4745].

In contrast to the success achieved with unsubstituted itaconate derivatives, asymmetric hydrogenation of β-substituted itaconic acid derivatives of general structure has been more challenging; relatively few reports of high enantioselectivity (over 90% ee) have appeared. No enantioselectivities above 90% ee have been reported for β-alkyl-substituted itaconates.

Itaconate derivatives that possess two substituents in the β-position have thus far proven impossible to hydrogenate with high enantioselectivities and high rates. The only reported example of this type revealed that dimethyl β,β-dimethylitaconate may be hydrogenated with a Rh-TRAP catalyst system with the highest enantioselectivities being 78% ee [R. Kuwano *et al, Tetrahedron: Asymmetry*, **1995**, *6*, 2521].

It should be noted that enantiomerically pure compounds are required for many applications in, for example, the pharmaceutical industry. Consequently, providing enantiomeric purity is the ultimate objective of an asymmetric process, and achieving high enantioselectivity in a transformation of the type described herein is crucial from a process standpoint. 90% ee is often selected as a lower acceptable limit, because compounds often may be purified to enantiomeric purity through recrystallisation when the initial value is above 90% ee. Enantiomeric excesses lower than 90% ee become increasingly more difficult to purify.

### Summary of the Invention

The present invention is based on the discovery that an efficient and high-yielding preparation of an enantiomerically enriched chiral carboxylic acid by asymmetric hydrogenation, in the presence of a rhodium complex of a chiral phosphine, is facilitated by use ofparticular salt forms ofthe hydrogenation substrate. Examples of such substrates are itaconates, which are referred to herein by way of example only. More generally, the products of the invention have the partial formula C-C-C-COOX, X being a cation. The corresponding acid will usually be obtained, on work-up.

The use of salt forms can have a number of advantages. Firstly, formation and isolation of a salt form, using a substantially stoichiometric amount of base, may provide a convenient means of effecting substrate purification prior to hydrogenation, should this be required. Secondly, at a given molar ratio of substrate to catalyst (S/C ratio) and reaction time, a higher substrate conversion and/or higher enantioselectivity can be achieved. Thirdly, high reaction rates allow reactions to be performed at low temperatures, e.g. 0°C, whereby higher product enantiopurity is observed.

### Description of the Invention

The substrate for hydrogenation is prochiral, i.e. it is asymmetrically substituted about the C=C bond. One substituent is -C-COOX, and the combination of chain length and carboxylate anion provides the ability of the substrate to coordinate a metal catalyst. There may be none or any substituents on the same C atom of the C=C bond as -C-COOX, provided that they do not interfere with the reaction. For example, in the hydrogenation of a substrate of the formula R³R⁴C=CR¹-CH₂-COOX, R¹, R³ and R⁴ are each essentially spectators, although R³ and R⁴ are not both hydrogen when R¹ is COOY. A characteristic of this invention is that no carboxylate function other than COOX is necessary.

Such substrates are known or may be prepared by methods known to those skilled in the art. In the particular case when R¹ is COOR², COOalkyl or COOaryl, both β-substituted and β,β-disubstituted itaconate derivatives may be prepared. Itaconates for use as substrates are also described in WO-A-9931041.

Suitable substrates for the hydrogenation process outlined above are ofthe general structure **7** or **8** (for the preparation of products **2**) 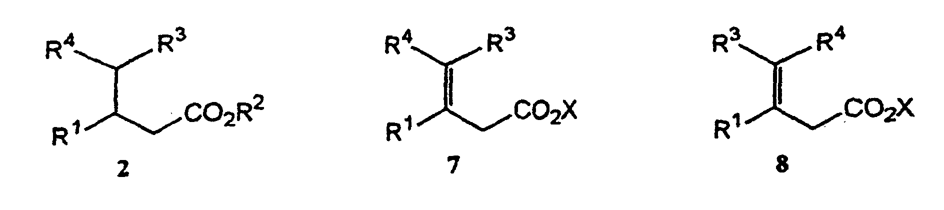 or a mixture thereof, wherein R¹, R³ and R⁴ can be independently H or an organic group of up to 30 C atoms, or R¹ is a cation or R³ and R⁴ are joined to form a ring, provided that at least one of R³ and R⁴ is not H, and R² is H or a cation X. In one embodiment, the invention provides an improved procedure in the case where one of R³ and R⁴ is H; typically, the other is C₁₋₂₀ alkyl or aralkyl. By way of example, the fact that β,β-disubstituted itaconates can be effectively hydrogenated in this process means also that R³ and R⁴ may each be an organic group of up to 30 C atoms, e.g. C₁₋₂₀ alkyl or aralkyl, and preferably the same, or may be linked to form a ring, e.g. a saturated carbocyclic ring. In this case, R¹ may be COOC₁₋₁₀ alkyl, COO aryl or COOaralkyl.

X may represent a metal, e.g. alkali metal, or other cation. The metal salt may be preformed or formed *in situ*, by introducing a strong base such as a metal alkoxide, e.g. NaOMe.

Alternatively, the salt may be formed with, for example, a counterion YH⁺ such as that derived from an amine Y or a phosphine Y. Primary C₁₋₁₀ alkylamines and cycloalkylamines are preferred, in particular, *tert*-butylamine. Tertiary amines such as triethylamine may also be used.

Especially when an amine or phosphine salt is used, it is usually isolated prior to use in the process, but alternatively may be generated *in situ*. Isolation of the precursor salt can be advantageous as a means of effecting substrate purification, usually by crystallisation, e.g. to remove any regioisomeric contaminants. However, this step is not always necessary, especially when the Stobbe condensation is carried out under carefully controlled conditions where regioisomeric contaminants are not formed, e.g. at a temperature of around 5°C rather than at normal room temperature.

Temperature effects may also be noted in the process of the present invention, with a lowering of reaction temperature resulting in improved enantioselectivities for certain substrates, e.g. when R³/R⁴ is a cyclic group, or if the precursor is an amine or phosphine salt. Especially in such cases, the reaction temperature may be less than 10°C, and is preferably -25 to +5°C.

Catalysts that are suitable for the asymmetric hydrogenation process comprise rhodium metal complexed to an appropriate chiral phosphine ligand. Preferably, the ligand is a monophosphine or diphosphine ligand which may be used in either enantiomeric form.

Preferred phosphines are those incorporating an appropriately substituted phosphorus heterocycle of general structure **10**, where n is zero or an integer 1 to 6, and where the carbocyclic framework of **10** is substituted with one or more R substituents such that the structure **10** is a chiral entity, and where the R substituent is an organic group of up to 20 C atoms, typically a C₁₋₁₀ linear or branched hydrocarbon substituent, but which also may contain heteroatoms. In the case where more than one R substituent is present in the structure **10**, these R substituents may be the same or different, and may be joined to form ring systems fused with the parent carbocyclic framework illustrated for **10**. Monophosphines containing the phosphorus heterocyclic unit **10** take the general structure **11**, where R' is an organic group of up to 20 C atoms. Alternatively, two phosphorus heterocycles of structure **10** may be tethered with a linking unit to form a diphosphine of general structure **12**, where the linking unit is an organic group of up to 30 C atoms, linear, branched or cyclic, hydrocarbon or heteroatomic in nature.

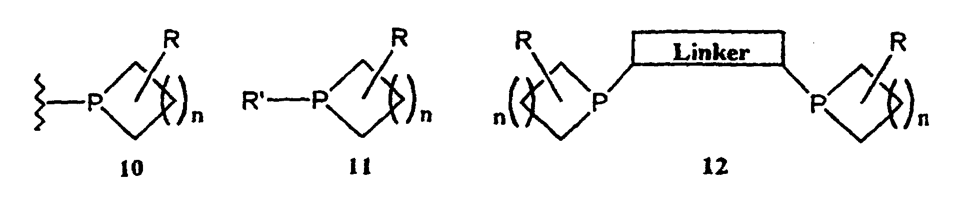

Examples of these ligands encompass 2,4-disubstituted phosphetanes **13**, e.g. as disclosed in WO-A-9802445, as well as the DuPHOS [US-A-5171892] and BPE [US-A-5008547] series of bisphospholanes, **14** and **15**, respectively. The latter ligands constitute the most preferred class of diphosphines for the asymmetric hydrogenation process described herein.

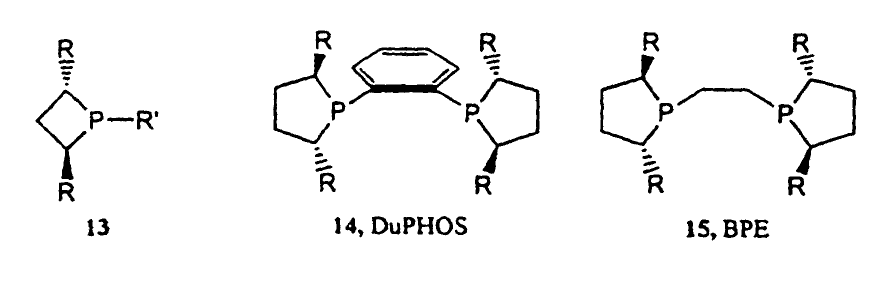

The possession of a series of homologous ligands of types 11-15 which are substituted with a range of different R groups is crucial for success in asymmetric hydrogenations since it is difficult to predict which catalyst will hydrogenate a particular substrate type with high selectivity. For a given substrate, enantioselectivities may be dependent upon the nature of the R-substituent attached to the carbocyclic ring of the DuPHOS, BPE or other ligand. Typically, a range of ligand-metal complexes may be screened, in order to identify the optimum catalyst for a given transformation, although such screening is readily done by one of ordinary skill in the art, if necessary with reference to the guidance provided herein. The appropriate complex may change, from substrate type to substrate type: rhodium complexes containing certain DuPHOS and BPE ligands have been shown to hydrogenate several types of olefinic substrates, such as enamides, with very high enantioselectivity [Burk et al., *J. Am. Chem. Soc*., **1993**, *115*, 10125], while other substrates such as α,β-unsaturated carboxylic acids and allylic alcohols are reduced with only very low selectivities. For example, both β-substituted and β,β-disubstituted α-enamide esters may be hydrogenated to α-amino acid derivatives with high enantioselectivity using certain DuPHOS and BPE-rhodium catalysts [Burk et al., *J. Am. Chem. Soc*., **1995**, *117*, 9375]. Furthermore, β-substituted α-arylenamides may be hydrogenated to α-arylalkylamine derivatives with high enantioselectivities [Burk et al., *J. Am. Chem. Soc*., **1996**, *118*, 5142], yet β,β-disubstituted α-arylenamides are hydrogenated with the same catalysts with very low enantioselectivity (0-5% ee).

The value of using a salt as the substrate is evident in the case where the hydrogenation substrate is a β,β-disubstituted itaconate derivative, for example wherein R³ =R⁴=methyl. Otherwise, it may be that high substrate conversion is difficult to achieve at acceptable S/C ratios (typically >200:1). See, for example, Examples 1 and 2. In the former, hydrogenation of the *tert*-butylamine salt of 2-isopropylidenesuccinic acid 1-methyl ester, catalysed by a rhodium(I) complex of (*R*,*R*)-methyl BPE, with S/C = 500: 1, was conducted at 0°C using methanol as solvent. This gave complete substrate conversion after 20 hours, to afford after salt cracking (*R*)-2-isopropylsuccinic acid 1-methyl ester in 95% ee. Enrichment of the salt to at least 99% ee could then be simply achieved by reslurrying in fresh solvent and then filtering. In Example 2, reaction of the free acid of of 2-isopropylidenesuccinic acid 1-methyl ester under similar conditions, with a higher catalyst loading (S/C = 300:1), gave only 33% substrate conversion, with (*R*)-2-isopropylsuccinic acid 1-methyl ester produced in 88% ee.

Overall, the present invention provides a straightforward process for the synthesis of valuable, highly enantiomerically enriched chiral carboxylic acid derivatives, starting from readily available, inexpensive starting materials.

The following Examples illustrate the invention, except Example 2 which is comparative.
TBME = *tert*-butyl methyl ether
GC = gas chromatographic analysis

### Example 1

**A.** A solution of *tert*-butylamine (124 mL, 1.19 mol) in *tert*-butyl methyl ether (TBME; 100 mL) was added dropwise, at room temperature, over a period of 2 hours, to a solution of 2-isopropylidenesuccinic acid 1-methyl ester (205 g, 1.19 mol) in TBME (350 mL). The resulting thick suspension was stirred at room temperature for an additional hour, then the solid precipitate was collected, washed with TBME (1 L) and dried under vacuum at 40°C for 48 hours to give 181 g of the salt as a white powder (yield: 62%).
**B.** A solution of the *tert*-butylamine salt of 2-isopropylidenesuccinic acid 1-methyl ester (162 g, 0.66 mol) in methanol (800 mL) was transferred to a 2 L high pressure hydrogenation vessel and degassed by pressurizing and venting four times with 10 bar of hydrogen. The vessel was then cooled to 0°C and a solution of [Rh(COD)(*S*,*S*)-Me-BPE]OTf (0.80 g, 0.0013 mol) in methanol (10 mL) was added through the solvent port. The reaction was purged again with hydrogen and stirred at 0°C under a pressure of hydrogen of 10-7 bar. After 22 hours, the temperature was allowed to raise to room temperature, the vessel was vented in a fume hood, the reaction mixture was transferred to a round-bottomed flask and the solvent was evaporated under reduced pressure. A sample (1 g) of the resulting solid residue was partitioned between HCl 2N (5 mL) and ethyl acetate (5 mL). The organic layer was dried over MgSO₄ and evaporated to give 2-(*S*)-isopropylsuccinic acid 1-methyl ester, ee 96% by GC. The bulk residue was suspended in ethyl acetate (600 mL) and stirred at room temperature for 48 hours, then collected and dried under vacuum to give 154 g of the *tert*-butylamine salt of 2-(*S*)-isopropylsuccinic acid monomethyl ester (yield: 94%). A sample (1 g) of the salt was worked up and analyzed as above, indicating an enantiomeric excess of 99% for the free acid.

### Example 2 (Comparative)

2-Isopropylidenesuccinic acid 1-methyl ester (0.86 g, 5.0 mmol) and sodium methoxide (0.10 g, 1.8 mmol) were placed in a 60 mL high pressure hydrogenation vessel and the vessel was purged with hydrogen (by pressurizing and venting three times with 10 bar of hydrogen). Methanol (9 mL, previously degassed by bubbling nitrogen for one hour at room temperature under stirring) was added through the solvent port and the vessel was then cooled to 0°C. A solution of [Rh(COD)(*R*,*R*)Me-BPE]OTf (0.010 g, 0.016 mmol, substrate/catalyst: 300/l) in methanol (1 mL) was added and the reactor was charged with 10 bar of hydrogen. The reaction was stirred at 0°C for 20 hours, then the solvent was evaporated under reduced pressure and the residue was partitioned between HCl 2N (20 mL) and ethyl acetate (20 mL). The organic layer was separated, dried over MgSO₄, evaporated to give a pale yellow oil. ¹H NMR analysis of the crude indicated that the reduced product and the starting material were present in a ratio 33 :67. The enantiomeric excess of 2-(*R*)-isopropylsuccinic acid 1-methyl ester was 88% by GC. This result shows that, for this particular substrate, the salt form used in Example 1 is preferable.

### Example 3

The *tert*-butyl ammonium salt of 2-isopropylidenesuccinic acid 1-methyl ester (0.80 g, 3.3 mmol) was prepared, and placed in a 60 mL high pressure hydrogenation vessel and the vessel was purged with hydrogen (by pressurizing and venting three times with 10 bar of hydrogen). Methanol (9 mL, previously degassed by bubbling nitrogen for one hour at room temperature under stirring) was added through the solvent port and the vessel was then cooled to 0°C. A solution of [Rh(COD)(*R*,*R*)Me-BPE]OTf (0.004 g, 0.0065 mmol, substrate/catalyst: 500/l) in methanol (1 mL) was added and the reactor was charged with 10 bar of hydrogen. The reaction was stirred at 0°C for 20 hours, then the solvent was evaporated under reduced pressure and the residue was partitioned between HCl 2N (20 mL) and ethyl acetate (20 mL). The organic layer was separated, dried over MgSO₄, evaporated to give a pale yellow oil. ¹H NMR analysis of the crude indicated that the conversion to the reduction product was more than 95%. The enantiomeric excess of 2-(*R*)-isopropylsuccinic acid 1-methyl ester was 95% by GC.

### Example 4

The *tert*-butylamine salt of (*E*)-2-(3-phenyl-2-propenylidene)succinic acid 1-methyl ester (1 g, 4.1 mmol) was prepared. This salt and [Rh(COD)(*R*,*R*)Me-DuPhos]BF₄ (6 mg, 0.01 mmol, substrate/catalyst: 400:1) were weighed in a 60 mL high pressure hydrogenation vessel and an atmosphere of nitrogen was introduced by evacuating the reactor and refilling with oxygen-free dry nitrogen. This procedure was repeated three times. Methanol (5 mL, previously degassed by bubbling nitrogen for one hour at room temperature while stirring) was added to the reactor through the solvent port. The reactor was charged with 690 kPa (100 psi) of hydrogen and the pressure released. The reactor was then repressurised to 965 kPa (140 psi) and the reaction was stirred for 16 hours, then the solvent was evaporate under reduced pressure and the residue was partitioned between HCl 2N (20 mL) and ethyl acetate (20 mL). The organic layer was separated, dried over MgSO₄, evaporated to give a pale yellow oil. ¹H NMR analysis of the crude indicated that the conversion to the reduction product was complete. The enantiomeric excess of 2-(S)-(3-phenyl-2-propenyl)succinic acid 1-methyl ester was 99% by GC.

### Example 5

The *tert*-butylamine salt of 2-cyclohexylidenesuccinic acid 1-methyl ester (0.91 g, 3.2 mmol) was prepared, and placed in a 60 mL high pressure hydrogenation vessel. The vessel was purged with hydrogen (by pressurizing and venting three times with 10 bar of hydrogen). Methanol (9 mL, previously degassed by bubbling nitrogen for one hour at room temperature under stirring) was added through the solvent port and the vessel was then cooled to 0°C. A solution of [Rh(COD)(R,R)Me-BPE]OTf(0.004 g, 0.0065 mmol, substrate/catalyst: 500/l) in methanol (1 mL) was added and the reactor was charged with 10 bar of hydrogen. The reaction was stirred at 0°C for 20 hours, then the solvent was evaporated under reduced pressure and the residue was partitioned between HCl 2N (20 mL) and ethyl acetate (20 mL). The organic layer was separated, dried over MgSO₄, evaporated to give 0.75 g of 2-cyclohexylsuccinic acid monomethyl ester as a pale yellow oil (yield 82%). The enantiomeric excess of 2-(R)-cyclohexylsuccinic acid 1-methyl ester was 96% by GC.

### Example 6

The *tert*-butylamine salt of 2-(2-adamantylidene)succinic acid 1-methyl ester (0.54 g, 1.6 mmol) was prepared, and placed in a 60 mL high pressure hydrogenation vessel. The vessel was purged with hydrogen (by pressurizing and venting three times with 10 bar of hydrogen). Methanol (9 mL, previously degassed by bubbling nitrogen for one hour at room temperature under stirring) was added through the solvent port and the vessel was then cooled to 0°C. A solution of [Rh(COD)(R,R)Me-BPE]OTf(0.004 g, 0.0065 mmol, substrate/catalyst: -250/l) in methanol (1 mL) was added and the reactor was charged with 10 bar of hydrogen. The reaction was stirred at 0°C for 23 hours, then the solvent was evaporated under reduced pressure and the residue was partitioned between HCl 2N (20 mL) and ethyl acetate (20 mL). The organic layer was separated, dried over MgSO₄, evaporated to give a pale yellow oil. ¹H NMR analysis of the crude indicated that the conversion to the reduction product was complete. The enantiomeric excess of 2-(*R*)-(2-adamantanyl)succinic acid monomethyl ester was 78% by GC. In similar experiments, carried out at room temperature (approx. 20°C), 2-(*R*)-(2-adamantanyl)succinic acid monomethyl ester was obtained with 63% ee.

### Example 7

**A.** A solution of *tert*-butylamine (0.91 g, 12.4 mmol) in *tert*-butyl methyl ether (TBME; 2 mL) was added dropwise, at room temperature, to a solution of 3,4-diphenyl-3-butenoic acid (12.4 mmol) in TBME (8 mL). The resulting thick suspension was stirred at room temperature for 15 minutes, then the solid precipitate was collected and dried under vacuum, to give the *tert*-butylamine salt of 3,4-diphenyl-3-butenoic acid (66 % yield) as a mixture of two geometric isomers in a 4:1 ratio (as determined by ¹H NMR analysis).
**B.** The *tert*-butylamine salt of 3,4-diphenyl-3-butenoic acid (0.31 g, 1 mmol, mixture of E/Z isomers) and [Rh(COD)(S,S)Me-BPE]OTf(6 mg, 0.01 mmol, substrate/catalyst: 100/l) were placed in a 60 mL high pressure hydrogenation vessel and the vessel was purged with hydrogen (by pressurising and venting three times with 10 bar of hydrogen). Methanol (10 mL, previously degassed by bubbling nitrogen for one hour at room temperature under stirring) was added through the injection port and the reactor was charged with 7 bar of hydrogen. The reaction was stirred at room temperature (approx 20°C) for 14 hours, then the solvent was evaporated under reduced pressure. ¹H NMR analysis of the crude indicated that the conversion to the reduction product was quantitative. The crude was then partitioned between HCl 2N (40 mL) and dichloromethane (40 mL). The organic layer was separated, dried over MgSO₄, evaporated to give a pale yellow oil (0.19 g, 83% yield). The enantiomeric excess of (R)-3,4-diphenylbutanoic acid was determined by chiral HPLC analysis to be 60%.

## Claims

1. A process for the preparation of an enantiomerically enriched chiral carboxylic acid derivative of formula 2 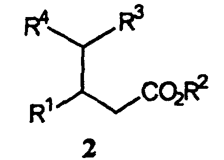 wherein R¹, R³ and R⁴ are each independently H or an organic group of up to 30 C atoms, or any two are linked to form a ring, provided that R³ and R⁴ are not both H, and R² is H or a cation, which comprises formation of a dehydro precursor salt of formula 7 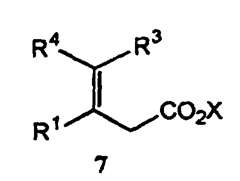 wherein X is a cation, optionally in the form of a mixture of such compounds when one of R³ and R⁴ is H, by reaction of a corresponding precursor acid with an at least substantially stoichiometric amount of base, and asymmetric hydrogenation of the salt in the presence of a rhodium complex of a chiral phosphine ligand having the partial formula 10 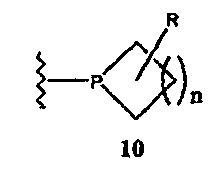 wherein n is 0 to 6 and R represents at least one non-hydrogen organic group of up to 30 C atoms; followed, if desired, by work-up to convert R² as a cation to R² as H.

2. A process according to claim 1, wherein R³ and R⁴ are each, or are linked to form, an organic group of up to 30 C atoms.

3. A process according to claim 2, wherein R³=R⁴.

4. A process according to claim 1, wherein one of R³ and R⁴ is H.

5. A process according to any preceding claim, wherein R³ and R⁴ are independently H, C₁₋₂₀ alkyl, aryl or aralkyl.

6. A process according to any preceding claim, wherein R¹ is COOC₁₋₁₀ alkyl or COOaralkyl.

7. A process according to any preceding claim, wherein the precursor is an amine or phosphine salt.

8. A process according to claim 7, wherein the precursor is an amine salt.

9. A process according to claim 8, wherein the amine is a primary amine.

10. A process according to claim 9, wherein the amine is *tert*-butylamine.

11. A process according to claim 8, wherein the amine is a tertiary amine.

12. A process according to claim 11, wherein the amine is triethylamine.

13. A process according to any of claims 1 to 6, wherein the precursor salt is a metal salt.

14. A process according to claim 13, wherein the metal is an alkali metal.

15. A process according to claim 14, wherein the metal is sodium or potassium.

16. A process according to any preceding claim, wherein the precursor salt is isolated prior to use in the asymmetric hydrogenation.

17. A process according to any of claims 1 to 15, wherein the precursor salt is generated *in situ*.

18. A process according to any preceding claim, wherein the ligand is of formula 11 or 12 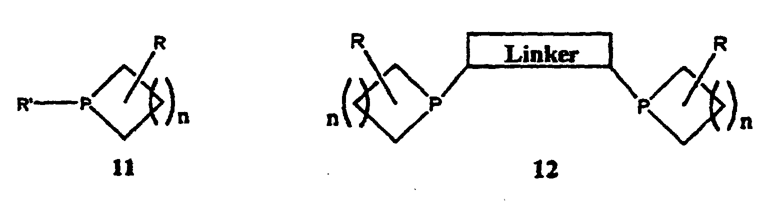 wherein Linker and R' are independently any non-hydrogen organic group of up to 30 C atoms.

19. A process according to claim 18, wherein the ligand is of formula 14 or 15

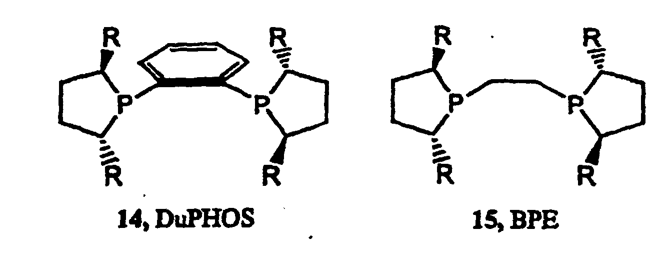

20. A process according to claim 19, wherein R is a C₁₋₈ linear or branched alkyl group, or an aromatic group.

21. A process according to any preceding claim, wherein the reaction temperature is less than 10°C.

22. A process according to any preceding claim, which gives the product in an enantiomeric excess of at least 90%.

## Patentansprüche

1. Verfahren zur Herstellung eines an Enantiomeren angereicherten chiralen Carbonsäurederivates der Formel 2: 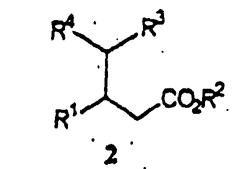 worin R¹, R³ und R⁴ jedes unabhängig H oder eine organische Gruppe mit bis zu 30 Kohlenstoffatomen sind oder zwei miteinander unter Bildung eines Ringes verbunden sind, vorausgesetzt daß R³ und R⁴ nicht beide H sind und R² = H oder ein Kation ist,
welches die Bildung eines Dehydrovorläuferssalzes der Formel 7 umfaßt: 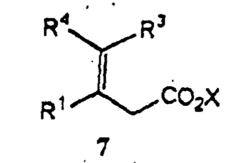 worin X ein Kation ist, wahlweise in Form einer Mischung solcher Verbindungen, wenn eines von R³ und R⁴ = H ist, durch Reaktion eines entsprechenden Vorläufers mit einer wenigstens im wesentlichen stöchiometrischen Menge von Base, und asymmetrische Hydrierung des Salzes in Anwesenheit eines Rhodiumkomplexes eines chiralen Phosphinliganden, der die Teilformel 10 hat: 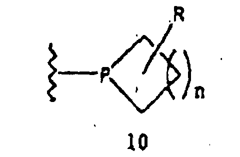 worin n = 0 bis 6 ist und R wenigstens eine organische Nicht-Wasserstoffgruppe mit bis zu 30 Kohlenstoffatomen darstellt, gewünschtenfalls gefolgt von Aufarbeitung zum Umwandeln von R² als ein Kation zu R² als H.

2. Verfahren nach Anspruch 1, bei welchem R³ und R⁴ jedes eine organische Gruppe sind oder verbunden sind unter Bildung einer organischen Gruppe mit bis zu 30 C-Atomen.

3. Verfahren nach Anspruch 2, bei welchem R³ = R⁴ ist.

4. Verfahren nach Anspruch 1, bei welchem eines von R³ und R⁴ = H ist.

5. Verfahren nach irgendeinem vorhergehenden Anspruch, bei welchem R³ und R⁴ unabhängig H, C₁₋₂₀-Alkyl, Aryl oder Aralkyl sind.

6. Verfahren nach irgendeinem vorhergehenden Anspruch, bei welchem R¹ = COOC₁₋₁₀-Alkyl oder COO-Aralkyl ist.

7. Verfahren nach irgendeinem vorhergehenden Anspruch, bei welchem der Vorläufer ein Amin- oder Phosphinsalz ist.

8. Verfahren nach Anspruch 7, bei welchem der Vorläufer ein Aminsalz ist.

9. Verfahren nach Anspruch 8, bei welchem das Amin ein primäres Amin ist.

10. Verfahren nach Anspruch 9, bei welchem das Amin tert-Butylamin ist.

11. Verfahren nach Anspruch 8, bei welchem das Amin ein tertiäres Amin ist.

12. Verfahren nach Anspruch 11, bei welchem das Amin Triethylamin ist.

13. Verfahren nach irgendeinem der Ansprüche 1 bis 6, bei welchem das Vorläufersalz ein Metallsalz ist.

14. Verfahren nach Anspruch 13, bei welchem das Metall ein Alkalimetall ist.

15. Verfahren nach Anspruch 14, bei welchem das Metall Natrium oder Kalium ist.

16. Verfahren nach irgendeinem vorhergehenden Anspruch, bei welchem das Vorläufersalz vor der Verwendung in der asymmetrischen Hydrierung isoliert wird.

17. Verfahren nach irgendeinem der Ansprüche 1 bis 15, bei welchem das Vorläufersalz in situ erzeugt wird.

18. Verfahren nach irgendeinem der Ansprüche 1 bis 15, bei welchem der Ligand Formel 11 oder Formel 12 entspricht, 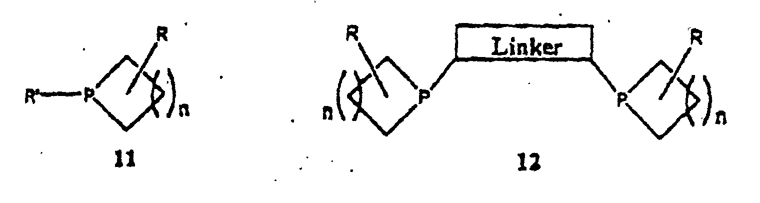 worin der Linker und R' unabhängig irgendeine organische Nicht-Wasserstoffgruppe mit bis zu 30 C-Atomen sind.

19. Verfahren nach Anspruch 18, bei welchem der Ligand der Formel 14 oder 15 entspricht:

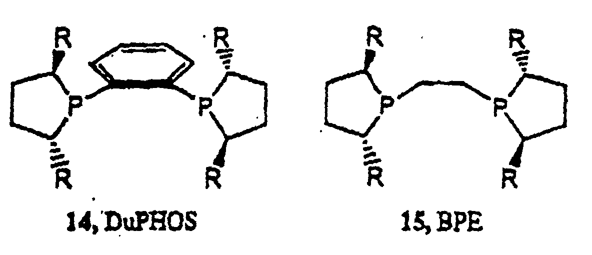

20. Verfahren nach Anspruch 19, bei welchem R eine lineare oder verzweigte C₁₋₈-Alkylgruppe oder eine aromatische Gruppe ist.

21. Verfahren nach irgendeinem vorhergehenden Anspruch, bei welchem die Reaktionstemperatur geringer als 10°C ist.

22. Verfahren nach irgendeinem vorhergehenden Anspruch, welches das Produkt in einem enantionmeren Überschuß von wenigstens 90% ergibt.

## Revendications

1. Procédé de préparation d'un dérivé d'acide carboxylique chiral, enrichi en l'un des énantiomères, de formule 2 : 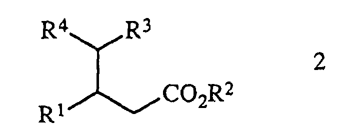 dans laquelle R¹, R³ et R⁴ représentent chacun indépendamment un atome d'hydrogène ou un groupe organique ayant jusqu'à 30 atomes de carbone, ou deux quelconques d'entre eux sont réunis pour former un noyau, étant entendu que R³ et R⁴ ne sont pas tous les deux des atomes d'hydrogène, et R² représente un atome d'hydrogène ou un cation, procédé qui comprend la formation d'un sel précurseur déhydro de formule 7 : 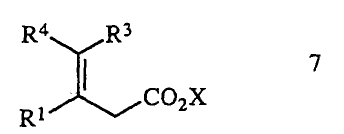 dans laquelle X représente un cation, éventuellement sous la forme d'un mélange de tels composés lorsque l'un de R³ et R⁴ représente un atome d'hydrogène, par réaction de l'acide précurseur correspondant avec une quantité au moins pratiquement stoechiométrique de base, et l'hydrogénation asymétrique du sel en présence d'un complexe du rhodium d'un ligand phosphine chiral de formule partielle 10 : 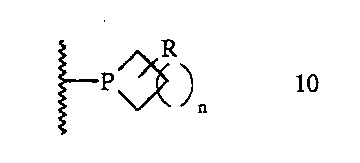 dans laquelle n est un nombre de 0 à 6 et R représente au moins un groupe organique ayant jusqu'à 30 atomes de carbone ou atomes autres que des atomes d'hydrogène, l'hydrogénation étant suivie, si on le souhaite, d'un traitement pour transformer le cation R² en atome d'hydrogène R².

2. Procédé selon la revendication 1, dans lequel R³ et R⁴ représentent chacun un groupe organique ayant jusqu'à 30 atomes de carbone ou sont réunis pour former un groupe organique ayant jusqu'à 30 atomes de carbone.

3. Procédé selon la revendication 2, dans lequel R³ et R⁴ sont identiques.

4. Procédé selon la revendication 1, dans lequel l'un de R³ et R⁴ est un atome d'hydrogène.

5. Procédé selon l'une quelconque des revendications précédentes, dans lequel R³ et R⁴ représentent chacun indépendamment un atome d'hydrogène ou un groupe alkyle, aryle ou aralkyle en C₁ à C₂₀.

6. Procédé selon l'une quelconque des revendications précédentes, dans lequel R¹ représente un groupe COO-alkyl(C₁ à C₁₀) ou un groupe COO-aralkyl.

7. Procédé selon l'une quelconque des revendications précédentes, dans lequel le précurseur est un sel d'amine ou de phosphine.

8. Procédé selon la revendication 7, dans lequel le précurseur est un sel d'amine.

9. Procédé selon la revendication 8, dans lequel l'amine est une amine primaire.

10. Procédé selon la revendication 9, dans lequel l'amine est la tert-butylamine.

11. Procédé selon la revendication 8, dans lequel l'amine est une amine tertiaire.

12. Procédé selon la revendication 11, dans lequel l'amine est la triéthylamine.

13. Procédé selon l'une quelconque des revendications 1 à 6, dans lequel le sel précurseur est un sel métallique.

14. Procédé selon la revendication 13, dans lequel le métal est un métal alcalin.

15. Procédé selon la revendication 14, dans lequel le métal est le sodium ou le potassium.

16. Procédé selon l'une quelconque des revendications précédentes, dans lequel le sel précurseur est isolé avant son utilisation dans l'hydrogénation asymétrique.

17. Procédé selon l'une quelconque des revendications 1 à 15, dans lequel le sel précurseur est produit *in situ*.

18. Procédé selon l'une quelconque des revendications précédentes, dans lequel le ligand est un ligand de formule 11 ou 12 : 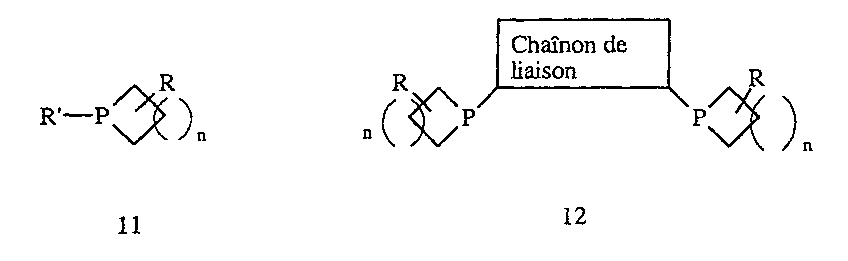 formules dans lesquelles le chaînon de liaison et R' représentent chacun indépendamment tout groupe organique ayant jusqu'à 30 atomes de carbone ou atomes autres que des atomes d'hydrogène.

19. Procédé selon la revendication 18, dans lequel le ligand est un ligand de formule 14 ou 15 :

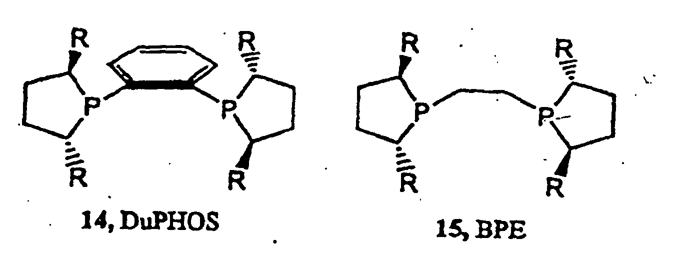

20. Procédé selon la revendication 19, dans lequel R représente un groupe alkyle, linéaire ou ramifié, en C₁ à C₈, ou un groupe aromatique.

21. Procédé selon l'une quelconque des revendications précédentes, dans lequel la température de réaction est inférieure à 10 °C.

22. Procédé selon l'une quelconque des revendications précédentes, qui donne le produit avec un excès de l'un des énantiomères correspondant à une proportion d'au moins 90 %.
